# EUROPÄISCHE PATENTSCHRIFT

(11) **EP 2 474 271 B1**
(45) Veröffentlichungstag und Bekanntmachung des Hinweises auf die Patenterteilung: **17.06.2015**
(21) Anmeldenummer: 12000078.1
(22) Anmeldetag: 09.01.2012
(51) Int. Cl.: A61B 17/04, A61B 17/064, A61B 17/128, A61B 17/17, A61F 2/46

(54) **Chirurgisches Instrument mit Sollbruchstelle**
Surgical instrument with frangible part
Instrument chirurgical avec un joint sécable

(30) Priorität: 07.01.2011 DE 102011000058; 05.01.2012 DE 102012100086
(43) Veröffentlichungstag der Anmeldung: 11.07.2012
(73) Patentinhaber: Z-Medical GmbH & Co. KG, 78532 Tuttlingen (DE)
(72) Erfinder: Combrowski, Zbigniew, 78532 Tuttlingen (DE); Paroth, Christel, 78532 Tuttlingen (DE); Henninger, Alexander, 78570 Mühlheim (DE)
(74) Vertreter: Arat, Dogan

(56) Entgegenhaltungen:
- EP-A1- 2 106 754
- EP-A2- 1 552 794
- EP-A2- 1 882 451
- WO-A1-03/030746
- WO-A1-2004/004578
- WO-A1-2010/127390
- WO-A2-2006/074414
- DE-A1- 19 859 952
- FR-A1- 2 731 610
- US-A1- 2010 280 621
- US-B1- 6 723 099

## Beschreibung

### Technisches Gebiet

Die Erfindung betrifft ein chirurgisches Instrument nach den Merkmalen des Oberbegriffs des Anspruchs 1.

### Stand der Technik

Aus dem Stand der Technik wird auf die WO 2004/004578 A1 hingewiesen. Dort ist ein Chirurgisches Instrument mit einem Implantat, einem Griffteil und einer Sollbruchstelle zwischen Implantat und Griffteil beschrieben, wobei dieses chirurgische Instrument den Oberbegriff des Anspruchs 1 beschreibt.

Weitere chirurgische Instrumente sind aus der EP 1 552 794 A2, der EP 2 106 754 A1, der US 6,723,099 B1 beschrieben, welche ebenfalls über ein Griffteil mit abbrechbarem Implantat verfügen.

Daneben wird auf die FR 2 731 610 A1, die EP 1 882 451 A2, die DE 198 59 952 A1 sowie die WO 03/030746A1 hingewiesen, welche ebenfalls relevante chirurgische Instrumente aufzeigen.

Der Vollständigkeit halber wird noch auf die US 2010/280621 A1, die WO 2010/127390 A1 und die WO 2006/074414 A2 hingewiesen, welche ebenfalls chirurgische Instrumente mit einem Griffteil aufzeigen, wobei das Griffteil über eine Sollbruchstelle mit einem Implantat verbunden ist.

Staples oder Klammern werden zur Verbindung von Knochenteilen, beispielsweise in der Hand- und Fusschirurgie nach Weil, Scarf, Austin, Chevron und Cheil und vielen weiteren OP Anwendungen eingesetzt. Die Klammern dienen zur Fixierung und/oder der Kompression von Knochenteilen und verbleiben in den meisten Fällen im Körper. Zufälligerweise sind diese Staples und/oder Klammern sehr kleine Implantate und bedingt durch Ihre Form schwer zu halten. Mit Einbringinstrumenten nach dem Stand der Technik sind Klammern und/oder Staples nur sehr zeitaufwendig einzusetzen. Ein weiteres Problem beim Implantieren von Staples und/oder Klammern ist, dass in der Regel vor dem Einsetzen der Klammer vorgebohrt werden wird. Die vorgebohrten Bohrlöcher sind nach dem Entfernen des Bohrers sehr schwierig aufzufinden. Aus dem Stand der Technik sind Bohrschablonen bekannt, allerdings können diese nicht in Kombination mit Staples verwendet werden.

### Beschreibung der Erfindung

Aufgabe der Erfindung ist es, ein chirurgisches Instrument so zu gestalten, dass die o.g. Nachteile behoben oder zumindest minimiert werden.

### Lösung der Aufgabe

Zur Lösung der Aufgabe führen die Merkmale des kennzeichnenden Teils des Anspruchs 1.

Bei einem chirurgischen Instrument mit einem Implantat und einem Griffteil sind das Implantat und das Griffteil über eine Sollbruchstelle verbunden. Das Implantat ist ein Fügeprofil. Fügeprofile sind bspw. Schrauben, Anker, Cages, Staples und/oder Klammern.

In typischen Ausführungsbeispielen umfasst das chirurgische Instrument ein Führungsrohr. Vorzugsweise weist das Führungsrohr einen Durchmesser kleiner oder gleich 4 mm auf. Besonders bevorzugt ist das Führungsrohr eine Kanüle.

Als Fügeprofil kommen hier verschiedene Formen in Betracht. Weitestgehend fallen hierunter in seitlicher Ansicht alle U-Profile, C-Profile, Halb- oder Dreiviertel- oder Teilkreisprofile, sowie die als Klammern oder Staples bezeichneten Implantate.

In typischen Ausführungsbeispielen ist das Implantat ein Staple. Staples eignen sich zur Fixierung von Knochenteilen und/oder Weichgewebe und/oder Bändern und/oder Sehnen.

Der Begriff "Staple" wird auch im Deutschen in der Medizintechnik als Fachbegriff für Fügeprofile verwendet. Die beiden Begriffe werden in der vorliegenden Anmeldung mit der gleichen Bedeutung verwendet.

Denkbar sind auch Fügeprofile mit noch mehr Schenkeln z.B. 4, 6 10 Schenkeln die auch in einer Art Stern, Wabenstruktur, Netzstruktur in beliebigen Formen und nicht nur flach, sondern auch an Knochenoberflächen angepasste Formungen haben können und eine Art Viertel, Halb, Dreiviertel oder beliebige Schalen mit Schenkeln bilden, die in den Knochen nach dem Vorbohren durch die integrierten Bohr- und Fixier- Schablonen in den Knochen eingeschlagen, eingepresst werden.

Da Fügeprofile sehr klein sind, sind diese schwer zu greifen und zu handhaben. Dadurch, dass das Griffteil im Vergleich zum Fügeprofil gut zu Greifen ist, ergibt sich der Vorteil, dass das Implantat zum Einbringen über das Griffteil komfortabel bedient, gehalten, aufgenommen, gedreht und/oder bewegt werden kann. Wenn das Implantat bzw. das Fügeprofil zumindest teilweise implantiert ist, kann das Griffteil ohne die Verwendung weiterer Werkzeuge an der Sollbruchstelle von dem Implantat bzw. dem Fügeprofil getrennt werden.

Der Griff kann aber auch aus mehreren Teilen bestehen, z.B. zweiteilig sein wobei das zweite Griffteil das nicht mit dem Fügeprofil verbunden ist, auch mit einer Sollbruchstelle zum Griffteil das mit dem Fügeprofil verbunden ist, abbrechbar ist.

Nach dem Abbrechen des zweiten Griffteils bildet diese eine sozusagen abnehmbare Bohrschablone für die Schenkel des Fügeprofils, und/oder Fixierbohrungen und/oder Führungsflächen und/oder Führungsnuten zum Führen des Fügeprofil beim Einbringen des Fügeprofils in den Knochen.

Das chirurgische Instrument umfasst eine Mehrzahl von Sollbruchstellen. Dadurch ergibt sich der Vorteil, dass das Griffteil und das Fügeprofil gut miteinander verbunden werden können. Ein weiterer Vorteil ist, dass zwischen den Sollbruchstellen Aussparungen vorgesehen sind. Besonders bevorzugt eigenen sich diese Aussparungen zum Eingriff eines später beschriebenen Einbringinstrumentes.

Fügeprofile, also auch Staples können aus einem implantierbaren resorbierbaren Material hergestellt sein.

Zweckmässigerweise wird das chirurgische Instrument einstückig, beispielsweise aus einer Titanplatte oder einem Blech hergestellt. Bevorzugte Herstellungsverfahren sind Laserschneiden, Drahterodieren, Stanzen, Biegen oder spanende Bearbeitungsverfahren wie Fräsen, Schleifen, möglich sind auch Staples aus implantierbaren resorbierbaren Materialien oder Kunststoffen. Dadurch ergibt sich der Vorteil, dass die Sollbruchstelle sehr genau gefertigt werden kann. Dies ist wichtig, um ein Brechen der Sollbruchstelle bei einem Einwirken von definierten Kräften und Drehmomenten zu gewährleisten. Ein weiterer Vorteil der genannten Herstellungsverfahren ist, dass das chirurgische Instrument mit Implantat relativ kostengünstig gefertigt werden kann.

In typischen Ausführungsbeispielen besteht das Fügeprofil bzw. der Staple aus Titandraht. Dadurch ergibt sich der Vorteil, dass das Fügeprofil sehr kostengünstig gefertigt werden kann.

Besonders bevorzugt ist die Sollbruchstelle bzw. die Mehrzahl der Sollbruchstellen so an dem Fügeprofil angeordnet, dass sie sich in einer Vertiefung des Fügeprofils befindet bzw. befinden und ein gratfreies Abbrechen möglich ist, bzw. der Grat tiefer als eine Oberfläche des Fügeprofils liegt. Dadurch ergibt sich der Vorteil, das das Fügeprofil bzw. ein möglicher Grat der Sollbruchstelle nicht zu Irritationen des Gewebes, der Haut, oder des Knochens führt.

In typischen Ausführungsbeispielen weist das Fügeprofil einen ersten Schenkel und einen zweiten Schenkel auf. Besonders bevorzugt umfasst das Fügeprofil einen Steg, geeignet, den ersten und den zweiten Schenkel zu verbinden.

Denkbar sind auch Fügeprofile mit noch mehr Schenkeln z.B. 4, 6 10 Schenkeln die auch in einer Art Stern, Wabenstruktur, Netzstruktur in beliebigen Formen und nicht nur flach sondern auch an Knochenoberflächen angepasste Formungen haben können und eine Art Viertel, Halb, Dreiviertelförmige oder beliebige Schalen mit Schenkeln bilden, die in den Knochen nach dem Vorbohren durch die integrierten Bohr- und Fixier-Schablonen in den Knochen eingeschlagen oder eingepresst werden.

In typischen Ausführungsbeispielen verlaufen der erste Schenkel und der zweite Schenkel des Fügeprofils parallel zueinander. In diesen Fällen schliessen die Schenkel und der Steg bevorzugt einen Winkel zwischen 120° und 15° ein. Besonders bevorzugte Ausführungsbeispiele der Fügeprofile sind sogenannte 90° Fügeprofile und sogenannte 26° Fügeprofile. Bei 90° Fügeprofilen schliessen der Steg und einer der Schenkel einen 90° Winkel ein. Bei 26° Fügeprofilen sind die parallelen Schenkel um 26° zum Steg abgewinkelt.

In typischen Ausführungsbeispielen schliessen die Schenkel der Fügeprofile Winkel zwischen 60° und 220° ein. Dadurch verlaufen die Schenkel nicht parallel zueinander. D.h. die Schenkel ragen voneinander ab oder laufen aufeinander zu. Dadurch ergibt sich der Vorteil, dass die einzelnen Schenkel Aufgaben, wie Kompression, Fixierung, Peilung und/oder Führung übernehmen können.

Auch ist eine Erhöhung der Kompression durch abgestufte Schenkel möglich und im bevorzugten Ausführungsbeispiel vorgesehen.

In typischen Ausführungsbeispielen weist der oder die Schenkel Vertiefungen, Nuten, Durchbrüche, Aussparungen, Schlitze und/oder Langlöcher auf. Dadurch ergibt sich der Vorteil, dass die Schenkel Aufgaben wie Fixierung oder Peilung übernehmen. Ein weiterer Vorteil ist, dass durch die spezielle Schenkelform das Fügeprofil bevorzugt federnd wirkt und sich eine Kompression ergibt. Vorteilhafterweise wird das spätere Verwachsen der Fügeprofile mit dem Knochen durch die o.g. Schenkelformen verbessert.

In typischen Ausführungsformen sind die freien Enden der Schenkel spitz und/oder mit einem Anschliff ausgebildet. Die Schenkel weisen Zacken und/oder Widerhaken auf. Dadurch ergibt sich der Vorteil, dass das Einbringen der Fügeprofile erleichtert wird und das Fügeprofil besser und sicherer im Knochen verankert wird.

Zweckmässigerweise umfasst das chirurgische Instrument eine Schlagfläche. Bevorzugt entsteht die Schlagfläche nach dem Abbrechen des Griffteils an dem Fügeprofil. Dadurch ergibt sich der Vorteil, dass das Fügeprofil nach dem Abbrechen des Griffteils mit einem weiteren Werkstück, mit dem auf die Schlagfläche eingewirkt wird, weiter in den Knochen bzw. das Gewebe eingetrieben werden kann.

In typischen Ausführungsbeispielen umfasst das Fügeprofil einen Aufbau. Bevorzugt bildet der Aufbau eine Schlagfläche aus. Dadurch ergibt sich der Vorteil, dass das Fügeprofil mit Griffteil, oder auch wenn das Griffteil bereits abgebrochen ist, über ein weiteres Werkzeug, mit dem auf die Schlagfläche geschlagen wird, in das Gewebe bzw. den Knochen eingetrieben werden kann.

Zweckmässigerweise umfasst das Griffteil eine Schlagfläche. Dadurch ergibt sich der Vorteil, dass das chirurgische Instrument über die Schlagfläche an dem Griffteil, bevorzugt durch ein weiteres Werkstück, in den Knochen bzw. das Gewebe eingetrieben werden kann.

Zweckmässigerweise ist die Schlagfläche des Griffteils ein Aufbau. Dadurch ergibt sich der Vorteil, dass das Griffteil und die Schlagfläche einstückig hergestellt werden können.

In typischen Ausführungsbeispielen weist das chirurgische Instrument eine Bohrung auf, wobei das Griffteil die Bohrung aufweist. Besonders bevorzugt weist das chirurgische Instrument eine Mehrzahl von Bohrungen auf.

Die Bohrungen dienen als Bohrschablone für die Schäfte des Fügeelements. Möglich ist es auch, weitere ausgewählte Bohrungen zur Fixierung der Knochen und der Bohrschablone einzubringen.

Dadurch ergibt sich der Vorteil, dass über die Bohrungen im Griffteil, das Griffteil an Bohrdrähten, die bereits im Körper eingebracht sind, positioniert werden kann. So kann das chirurgische Instrument bzw. das Fügeprofil gegenüber den Bohrdrähten oder bereits bestehenden Bohrungen positioniert werden.

In anderen Beispielen ist der Staple beziehungsweise das Implantat rohrförmig oder teilweise rohrförmig ausgebildet, z.B. als rundes oder mehreckiges Profil. Zweckmässigerweise sind diese bevorzugt aus einem Rohr oder Vollmaterial bevorzugt rund hergestellt. Bevorzugte Herstellungsverfahren sind Lasern oder Spritzverfahren, wobei auch hier alle Varianten möglich sind.

In typischen Ausführungsbeispielen umfasst das Implantat, insbesondere der Staple, Spikes. Bevorzugt sind die Spikes zwischen den Schenkeln des Staple angeordnet. Durch die Spikes ergibt sich der Vorteil, dass mit diesen zusätzlich auch Weichgewebe, Sehnen oder Bänder z.B. am Knochen fixiert werden können.

In anderen Beispielen sind sehr kleine Staple-Ausführungen aus rohrförmigen oder teilweise aus rohrförmigem Material, wie oben beschrieben, hergestellt. Diese werden vorzugsweise mittels eines Führungsrohres oder einer Kanüle mit einem Durchmesser kleiner als 4mm in den Körper eingebracht. Diese haben den Vorteil, dass die Staples mittels eines "mini open"-Verfahrens oder perkutan durch einen gezielten Zugang eingesetzt werden können. Dadurch ergibt sich der Vorteilt, dass das Verfahren zum Verbinden eines Risses in einem Meniskus eingesetzte werden kann. Dies ist schneller und einfacher als ein Verfahren mit mehreren Ankern und einem Faden.

In anderen Beispielen umfasst das Implantat, insbesondere der Staple eine seitliche Lasche mit einer Bohrung. Dadurch ergibt sich der Vorteil, dass eine zusätzliche Sicherung des Staples mit einer Schraube möglich ist.

In typischen Ausführungsbeispielen umfasst das Implantat, insbesondere der Staple mindestens einen Keil und/oder eine Lasche mit einer Bohrung zwischen den Schenkeln als festen Abstandshalter, wobei der Keil eine feste Winkelkorrektur oder einen festen Abstand vorgibt.

In typischen Ausführungsbeispielen umfasst der Staple eine Aussparung und/oder eine Bohrung. Vorzugsweise kann in diese ein Instrument eingesetzt werden. Dadurch ergibt sich der Vorteil, dass der Staple nach dem Verheilen oder bei einer Revision leichter wieder entfernt werden kann. Vorzugsweise dienen die Aussparungen oder die Bohrungen als Sollbruchstelle, so dass der Anwender diese gezielt z.B. mit einem Seitenschneider durchtrennen kann. Vorteil ist, dass zur Entnahme dieser durchtrennten Stücke nur kleine Zugänge gemacht werden müssen, was ästhetischer ist und schneller verheilt.

In typischen Ausführungsbeispielen umfasst eine Bohrschablone mindestens einen Schlitz, z. B. einen V-Schlitz oder einen Z-Schlitz mit verschiedenen Winkeln. Vorzugsweise ist die Bohrschablone als Sägeschablone ausgebildet mit einem seitlichen Anschlag für das Sägeblatt. Alternativ weist das chirurgische Instrument eine abbrechbare separate Sägeschablone am Griff mit mindestens einer Fixierbohrung und/oder Navigationshilfe auf.

In anderen Beispielen umfasst der Staple bzw. das Implantat eine Mehrzahl von Schenkeln. Vorzugsweise umfasst der Staple eine Platte, insbesondere eine Kompressionsplatten zwischen den Schenkeln. Vorzugsweise weist die Platte ein Mehrzahl von Bohrungen auf. Besonders bevorzugt dienen die Bohrungen zur Fixierung mit Bohrdrähten und/oder zur Verschraubung der Knochenteile. Vorteil dieser Ausführung gegenüber der Fixierung von Knochenteilen mit Platten und Nägeln und/oder Schrauben ist, dass bei dem Einsatz eine Stapels mit Schenkeln weniger Schrauben gebraucht werden und durch das Schenkeldesign eine anhaltende Kompression erreicht wird, was bei Platten kaum möglich ist. Des weiterein ist Vorteilhaft, dass ein Staple schneller zu montieren und auch schneller wieder auszubauen ist.

In anderen Beispielen ist das Plattendesign bevorzugt der Knochenoberflächen angepasst. Dadurch ergibt sich der Vorteil, dass unterschiedliche Anforderungen an den Knochenbau erfüllt werden können. Beispielsweise sind Vorzugsweise ist die Platte je nach Anwendungsfall als gerade oder annähernd gerade Platte ausgebildet.

In anderen Beispielen ist das Implantat als annähernd gerade Platte und zusätzlich abgewinkelt und/oder gebogen ausgebildet, um Winkeln und Radien ausgleichen zu können oder als Platte mit Stufen, insbesondere zum Ausgleichen von Höhenunterschieden.

In anderen Beispielen ist an die Platte mindestens ein Keil angeformt. Vorzugsweise ist der Keil zwischen den Schenkeln angeordnet. Dadurch ergibt sich der Vorteil, dass der Keil eine feste Winkelkorrektur oder einen festen Abstand vorgibt.

In typischen Ausführungsbeispielen umfasst das chirurgische Instrument eine abbrechbare Rohr- und/oder Sägeschablone. Vorzugsweise umfasst diese eine Fixierungsbohrung und/oder eine Navigationshilfe.

In anderen Beispielen ist das Implantat ein Anker. Anker werden vorzugsweise aus rundem Vollmaterial, Röhrchen und in flachen Formen aus allen medizinisch zugelassenen Materialien hergestellt. Meist weisen sie an den Mantelflächen gewindeartige oder jegliche Arten von Verzahnungen auf, um das Implantat sicher im Knochen zu verankern. Auch sind dübelartige Ausführungen möglich, die nach dem Einbringen expandieren. Dadurch ergibt sich der Vorteil, dass der Anker auf einfache Art und Weise eingebracht werden kann. Bis jetzt bestand beim Einbringen von Ankern immer das Problem der sicheren Befestigung der Bänder, aber auch der Komplexität und Bedienbarkeit der dazugehörigen Setzinstrumente.

Des weiteren ist vorteilhaft, dass das chirurgische Instrument mit einem Anker als Implantat einstückig und damit günstig herstellbar sind. Dies ist insbesondere bei einem Einwegprodukt ein immenser Vorteil wenn alles OP relevante steril verpackt bereitgestellt werden kann, und keine aufwendigen, sondern - wenn überhaupt - nur noch sehr schlanke Instrumentensets benötigt werden.

In anderen Beispielen ist der Anker aus einem runden, teilweisen Vollmaterial oder einem Röhrchen hergestellt. Vorzugsweise ist der Anker dübelartig und/oder in flachen Formen hergestellt. Zur Herstellung eignen sich alle medizinisch zugelassenen Materialien.

In anderen Beispielen weisen die Mantelflächen ein Gewinde und/oder eine Verzahnung auf. Dadurch ergibt sich der Vorteil, dass der Anker sicher im Knochen befestigt werden kann.

In anderen Beispielen umfasst das chirurgische Instrument als Implantat einen Doppelanker. Dadurch ergibt sich der Vorteil, dass beispielsweise ein Band in einer Vorrichtung mit einer bestimmte Spannkraft vorgespannt werden kann und dann in einem definierten Abstand in zwei Knochenteile eingesetzt werden kann. Vorzugsweise sind hierzu an dem Anker und oder Schaft ebenso wie bei allen anderen Varianten auch Fixierbohrungen, Querfixierbohrungen, eine Bohr-, eine Sägeschablone sowie eine Navigationseinrichtung integriert.

In anderen Beispielen weist der Anker einen Ankerschenkel aus Kunststoff oder resorbierbarem Material auf. Besonders bevorzugt ist ein Verbindungssteg an den Anker Angespritzt. Dadurch ergibt sich der Vorteil, dass auf ein zusätzliches Nahtmaterial verzichtet werden kann.

Vorzugsweise weist jeder Ankerschenkel einen abtrennbaren Verlängerungsschaft auf. Dadurch ergibt sich der Vorteil, dass eine Mehrzahl von Ankern zusammengefaltet, zusammengeklappt, nebeneinander und/oder hintereinander wie in einem Magazin eingeordnet werden können. Dies ist vorteilhaft, weil die Anker so aus einer Kanüle als Magazin insbesondere zum "Nähen" von Rissen eingesetzt werden können. Vorteilhaft ist auch, dass keine aufwändige Knotentechnik eingesetzt werden muss. Die ganze Operation ist mit einem Instrument und einer Implantatart möglich

In anderen Beispielen ist das Implantat eine Klammer. Klammern werden in verschiedenen Grössen bei offen- und bei minimalinvasiven-Operationen zur Abbindung von blutführenden Gefässen, Därmen und Organen eingesetzt. Die Hohlschaft-Halteinstrumente hierfür weisen meistens einen Durchmesser von 5 mm bis 12 mm.

Meistens werden mehrere Klammern beidseitig an der Stelle hintereinander gesetzt, wo auch z.B. ein Organ durchtrennt werden soll.

Vorzugsweise werden Klammern werden in teilweise runden, teilweise eckigen, und in andern Formen aus allen medizinisch zugelassenen Materialien hergestellt. Bevorzugt umfasst die Klammer ein Schloss, in das die beide Klammerschenkel verrasten, wenn diese zusammengedrückt werden. Das Einrasten geschieht bevorzugt bei Erreichen einer bestimmten Schliesskraft. Vorzugsweise sind die Klammern aus Kunststoff hergestellt.

In anderen Beispielen sind die Klammern aus Titan hergestellt. Vorzugsweise sind die Materialeigenschaften so gewählt, dass die Klammer nach dem Zusammendrücken die Form behält.

Die Problematik ist, dass die Klammern sehr kleine Bauteile sind. Das Entnehmen aus dem Magazin und das Halten im Instrumenten-Maulteil bis zur OP-Stelle ist schwierig, weil insbesondere bei den Ausführungen aus Titan keine Sicherung gegen das Herausfallen integrierbar ist.

In anderen Beispielen umfasst das Griffteil eine Kupplung, geeignet zur Verbindung mit einem Griff. Dadurch ergibt sich der Vorteil, dass jeder Anwender sein bevorzugtes Griffteil anschliessen kann.

In anderen Beispielen ist ein Griff einstückig an das Griffteil angeformt. Dadurch ergibt sich der Vorteil, dass das chirurgische Instrument auf einfache Art und Weise herstellbar ist und als Einheit, steril verpackt angeboten werden kann.

In anderen Beispielen umfasst die Klammer einen ersten Schaft und einen zweiten Schaft. Vorzugsweise weist der Schaft einen Durchmesser kleiner als 5 mm, besonders bevorzugt kleiner 3,5mm auf. Dadurch ergibt sich der Vorteil, der mehrere Schaftinstrumente durch eine kleine Öffnung, insbesondere den Bauchnabel eingesetzt werden können.

In anderen Beispielen ist der Schaft abgestumpft ausgebildet. Dadurch ergibt sich der Vorteil, das kein Gewebe verletzt wird.

In anderen Beispielen ist der Schaft teilweise abgewinkelt und/oder in einem Radius gebogen. Dadurch ergibt sich der Vorteil, dass die Klammer an einer abtrennbaren Verlängerung angebracht ist, wobei die Verlängerung je nach Anwendungsfall flexibel gestaltet werden kann.

In anderen Beispielen ist der Schaft aus einem flexiblen und biegbaren Material hergestellt. Dadurch ergibt sich der Vorteil, dass der Anwender vor dem Setzen der Klammer den Schaft in eine beliebige Krümmung biegen kann, um besser an die OP-Stelle zu kommen, ohne neue Zugänge setzen zu müssen.

In anderen Beispielen ist die Schaftform so ausgestaltet, dass eine Mehrzahl von Klammern, bevorzugt bis zu sechs Klammern gleichzeitig bereitstehen, die nacheinander abgeschlossen und/oder gesetzt werden können.

In anderen Beispielen ist die Kupplung nicht im Schaft angeordnet, sondern ausserhalb des Schaftes. Dadurch ergibt sich der Vorteil, dass ein Lademechanismus für die Klammern als Implantate grösser gebaut werden kann.

Vorzugsweise sind die Mehrzahl der Klammern miteinander verbunden. Bevorzugt sind die Klammern seitlich in Einbringrichtung miteinander verbunden. Alternativ sind die Klammern hintereinander angeordnet und miteinander verbunden. Dadurch ergibt sich der Vorteil, dass die Klammern einfacher in einem Magazin aufgenommen werden können.

Vorzugsweise besteht die Klammer aus einem Materialverbund. Vorzugsweise ist zumindest eines der Materialien resorbierbar. In typischen Ausführungsbeispielen umfasst die abtrennbare Verlängerung eine Kupplung zur Aufnahme in einem Griffteil.

Im anderen Beispielen ist das Implantat ein Cage. Ein solcher Cage ist beispielsweise in der DE 10 2011 002 076 offenbart. Vorzugsweise werden Cages zu Stabilisierung und Beabstandung von Wirbelkörpern eingesetzt. Wichtig ist jedoch, dass diese Cages besonders geeignet für die folgenden Einbringverfahren sind: "mini-open" und perkutan. Dazu umfasst das chirurgische Instrument vorzugsweise eine Führungsrohr, insbesondere eine Dadurch ergibt sich der Vorteil, dass der Cage in einem bestimmten Winkel aufstellbar ist, besonders gut navigiert werden kann und parallel expandiert werden kann. Diese Cageausführung kann auch in anderen medizinischen Bereichen, wie bspw. zur Hohlraumschaffung für die Kryphoplastie, tiermedizinischen und nicht medizinischen Bereichen angewendet werden.

Vorzugsweise umfasst der Cage eine Oberschale und eine Unterschale. Besonders bevorzugt sind die Oberschale und die Unterschale über eine Verstelleinrichtung verschiebbar zueinander verbunden. Vorzugsweise ist als Verstelleinrichtung ein Keil eingesetzt. Dadurch ergibt sich der Vorteil, dass ein Abstand der Oberschale zu der Unterschale durch einfaches Verschieben des Keils verändert werden kann.

In anderen Beispielen wird als Verstelleinrichtung ein Keil, eine Spindel, ein Luftkissen, ein Gelkissen oder ein Band eingesetzt. Dadurch ergibt sich der Vorteil, dass der Cage zur dynamischen Verbindung von Wirbelkörpern genutzt werden kann.

Ein Griffteil ist über zumindest eine Sollbruchstelle mit dem Cage als Implantat verbunden.

Bevorzugt ist mindestens die Unter- und/oder Oberschale und/oder z.B. der Keil zusätzlich mit mindestens einer Platte mit mindestens einer Befestigungsbohrung ausgestattet, um die Wirbelkörper zu versteifen. Bevorzugt greifen beide Plattenseiten formschlüssig ineinander, um höhere Kräfte aufnehmen zu können. Alternativ kann auch eine Verriegelungsplatte zusätzlich aufgeschraubt werden oder auch mit einer abtrennbaren Verbindung integriert werden.

Bevorzugt ist mindestens die Unter- und/oder Oberschale und/oder z.B. der Keil zusätzlich mit einem Stapleschenkel oder einer Stapleschenkelplatte mit Befestigungsbohrungen ausgestattet, um die Wirbelkörper zu versteifen, wobei bevorzugt beide Stapleschenkelseiten formschlüssig ineinandergreifen, um höhere Kräfte aufnehmen zu können. Alternativ kann auch eine Verriegelungsplatte zusätzlich aufgeschraubt werden oder auch mit einer abtrennbaren Verbindung integriert werden.

In anderen Beispielen werden an der Verbindungsstelle zwischen Implantat und Griffteil, vorzugsweise vor dem Abbrechen des Griffteils weitere Aufgaben wie Einbringen, Navigieren, Herstellern, und Fixieren des Cages ausgeübt.

In anderen Beispielen umfasst der Cage teilweise Auflageflächen. Vorzugsweise sind die Auflageflächen geeignet, aufgebrachte Druckkräfte beim Abreissen oder Einschlagen des Cages aufzunehmen.

In anderen Beispielen umfasst das Instrument an einem Ende eine Kupplung, geeignet zur Verbindung mit einem Einbringinstrument. Dadurch ergibt sich der Vorteil, dass die Herstellung kostengünstig möglich ist.

Alternativ kann das Instrument endseitig mit einem Griff verbunden sein. Vorzugsweise sind Griff und Implantat aus einem Material hergestellt. Vorzugsweise werden als Herstellverfahren Laserschneiden, Erodieren, Zerspannen oder Spritzen eingesetzt.

Nach dem Einsetzen des Cages bspw, in ein Bandscheibenfach kann dieser über die Verbindungen zur Navigation und ausgeübten Zug auf diese in beliebiger Richtung seitlich in einem bestimmten Winkel gedreht werden. Hierzu kann am Griff eine nicht dargestellte Skala angebracht sein, die es dem Anwender ermöglicht, die gewünschte Winkelstellung zu kontrollieren und/oder zu halten.

In anderen Beispielen umfasst das Instrument einen Anschlag oder eine Raste. Dadurch ergibt sich der Vorteil, dass ein Anwender erkennt und/oder spürt, ob die gewünschte Winkelposition erreicht ist und der Cage expandiert werden kann.

In anderen Beispielen ist die Sollbruchstelle so ausgelegt, dass das Abbrechen und/oder Abtrennen der Verbindung nach dem Expandieren des Cages bevorzugt bei Erreichen einer definierten Kraft oder eines definierten Drehmoments geschieht. Vorzugsweise wird erst anschliessend das Griffteil abgetrennt.

Vorzugsweise ist der Keil so ausgebildet, dass, wenn beim Expandierenden des Cages eine bestimmte Position nicht erreicht wird, der höchste Punkt des Keils übersprungen wird. Dadurch ergibt sich der Vorteil, dass der Cage wieder zusammengefahren wird und so besser entnommen werden kann.

Vorzugsweise ist die Sollbruchstelle so ausgestaltet, dass der Vorgang des Abtrennens eine definierte Bewegung auslösen kann. Beispiele für diese Bewegungen können eine Drehbewegung oder eine lineare Bewegung sein. Unter Umständen kann auch Werkzeug beispielsweise in Form eines Meisels oder einer Zange zum Durchtrennen der Sollbruchstelle herangezogen werden.

Vorzugsweise umfasst das Instrument ein Band, geeigneten zum Verbinden der abzubrechenden Teile. Dadurch ergibt sich der Vorteil, dass die abgebrochenen Teile kontrolliert werden können, um zu verhindern, dass diese umliegendes Gewebe beschädigen, verletzen und/oder verloren gehen.

In anderen Beispielen ist das Instrument aus einer Mehrzahl von Werkstoffen gefertigt. Dadurch ergibt sich der Vorteil, dass alle Elemente aus dem Material mit den günstigsten Eigenschaften für ihre Funktion hergestellt werden können.

Im anderen Beispielen umfasst das Instrument eine Stange, geeignet zum Ausüben einer Navigierbewegung. Vorzugsweise ist die Stange mit dem Cage verbindbar. Besonders bevorzugt umfasst die Stange als Material eine Formgedächtnislegierung, insbesondere Nitinol oder ist als eine Schnur ausgebildet, geeignet den Cage über Zugbewegungen zu navigieren. Besonders bevorzugt weist der Cage Bohrungen oder Haltepunkte auf. Dadurch ergibt sich der Vorteil, dass die Schnur oder eine Stange auf einfache Art und Weise festgelegt werden kann.

Bevorzugt umfasst das Einbringinstrument einen Griff. Dadurch ergibt sich der Vorteil, dass das Einbringinstrument für einen Chirurgen gut bedienbar und zu handhaben ist.

Zweckmässigerweise umfasst das Einbringinstrument eine Führungsnut für das chirurgische Instrument mit Implantat. Bevorzugt wird das Griffteil in der Führungsnut geführt. Zweckmässigerweise kann auch das Fügeprofil in der Führungsnut geführt werden. Besonders bevorzugt eignet sich die Führungsnut zum Führen des Griffteils und anschliessend zum Führen der Fügeprofile. Noch bevorzugter umfasst das Einbringinstrument zwei oder eine Mehrzahl von Führungsnuten. Dadurch ergibt sich der Vorteil, dass das chirurgische Instrument sicher mit dem Einbringinstrument geführt werden kann.

In typischen Ausführungsbeispielen umfasst das Einbringinstrument eine Schlagfläche. Dadurch ergibt sich der Vorteil, dass mit dem Einbringinstrument das chirurgische Instrument auch in hartes Gewebe, wie Knochen eingebracht werden kann.

Zweckmässigerweise umfasst das Einbringinstrument eine Auflagefläche. Bevorzugt ist die Auflagefläche mit der Schlagfläche der Fügeprofile und/oder der Schlagfläche des Griffteils in Wirkverbindung bringbar. Dadurch ergibt sich der Vorteil, dass Schläge, die auf die Schlagfläche des Einbringinstruments aufgebracht werden, sicher und geführt und vor allem im richtigen Winkel auf das chirurgische Instrument, insbesondere das Griffteil und/oder das Fügeprofil übertragen werden können. Dadurch ergibt sich der Vorteil, dass verhindert wird, dass das Griffteil von dem Fügeprofil beim Aufbringen von Schlägen auf die Schlagfläche des Einbringinstruments abbricht.

Zweckmässigerweise umfasst die Auflagefläche eine Führungsnut. Dadurch ergibt sich der Vorteil, dass das chirurgische Instrument sicher an der Auflagefläche des Einbringinstruments anliegt und geführt werden kann.

Die Bohrschablone umfasst eine Schablone mit Griff, geeignet zum Einbringen von Bohrungen, in die das Fügeprofil des chirurgischen Instruments eingebracht wird. Dadurch ergibt sich der Vorteil, dass das Vorbohren von Öffnungen für das Fügeprofil erleichtert wird.

Die Bohrschablone umfasst eine Schablone, geeignet zum Einbringen von Bohrungen, zur Fixierung des Knochens. Bevorzugt werden in die Bohrungen Bohrdrähte eingebracht. Dadurch ergibt sich der Vorteil, dass das chirurgische Instrument über die Bohrschablone und mittels der in der Bohrschablone eingebrachter Bohrdrähte relativ leicht zu bereits vorgebohrten Bohrungen zum Einbringen des Fügeprofils positioniert werden kann. Dadurch wird das Auffinden der Bohrungen für das Fügeprofil erleichtert.

Die Bohrschablone eine oder mehrere Führungsfläche(n) und/oder Führungsnute(n) zum Führen des chirurgischen Instruments. Bevorzugt eignet sich die Führungsfläche zum Führen des Fügeprofils. Besonders bevorzugt eignet sich die Führungsfläche zum Führen des Griffteils beim Einbringen der Fügeprofile. Dadurch ergibt sich der Vorteil, dass das Fügeprofil geführt und in einem definierten Winkel in das Gewebe eingebracht werden kann.

### Kurze Beschreibung der Zeichnungen

Nachfolgend wird die Erfindung anhand der beiliegenden Figuren kurz beschrieben, wobei die Figuren zeigen:
- Figur 1: zeigt eine Vorderansicht eines chirurgischen Instruments mit einem Griffteil und eines Fügeprofils;
- Figur 2: zeigt eine perspektivische Darstellung des chirurgischen Instruments gemäss Figur 1;
- Figur 3: zeigt eine Vorderansicht eines weiteren Ausführungsbeispiels eines chirurgischen Instruments mit einem Griffteil und eines Fügeprofils;
- Figur 4: zeigt eine perspektivische Darstellung des chirurgischen Instruments gemäss Figur 3;
- Figur 5: zeigt eine Vorderansicht eines Einbringinstruments mit einem chirurgischen Instrument;
- Figur 6: zeigt eine perspektivische Darstellung des Einbringinstruments ohne das chirurgische Instrument;
- Figur 7: zeigt eine perspektivische Darstellung einer Bohrschablone;
- Figur 8: zeigt eine Vorderansicht der Bohrschablone nach Figur 7;
- Figuren 9 bis 12: zeigen vergrösserte Ausschnitte der Bohrschablone nach Figur 8;
- Figur 13: zeigt eine perspektivische Darstellung des chirurgischen Instruments mit einem Einbringinstrument und einer Bohrschablone beim Einbringen des chirurgischen Instruments in Gewebe;
- Figur 14: zeigt eine schematische Darstellung einer Vorderansicht eines chirurgischen Instruments mit einem Staple als Implantat;
- Figur 15: zeigt eine schematische Darstellung eines weiteren Beispiels eines chirurgischen Instruments mit einem Staple als Implantat und einer Kanüle;
- Figur 16: zeigt eine schematische Darstellung einer Draufsicht auf das chirurgische Instrument nach Figur 15;
- Figur 17: zeigt eine schematische Darstellung einer perspektivischen Ansicht des Beispiels des chirurgischen Instruments nach den Figuren 15 und 16;
- Figur 18: zeigt eine schematische Darstellung einer Seitenansicht eines weiteren Beispiels eines chirurgischen Instruments mit einem Staple mit Spikes als Implantat;
- Figur 19: zeigt eine schematische Darstellung einer Draufsicht auf das chirurgische Instrument nach Figur 18;
- Figur 20: zeigt eine perspektivische Darstellung des chirurgischen Instruments nach den Figuren 18 und 19;
- Figur 21: zeigt eine schematische Darstellung einer Seitenansicht eines weiteren Ausführungsbeispiels eines chirurgischen Instruments, wobei das Implantat ein Staple mit vier Schenkeln ist;
- Figur 22: zeigt eine schematische Darstellung einer perspektivischen Ansicht des chirurgischen Instruments mit einem Staple mit vier Schenkeln nach Figur 21;
- Figur 23: zeigt eine schematische Darstellung einer Seitenansicht eines weiteren Beispiels eines Instruments mit einem Staple mit einer Stapleplatte und einem seitlichen Keil zur Winkelkorrektur;
- Figur 24: zeigt eine schematische Darstellung einer perspektivischen Ansicht des chirurgischen Instruments nach Figur 23;
- Figur 25: zeigt eine schematische Darstellung einer Seitenansicht eines weiteren Beispiels eines chirurgischen Instruments, bei dem das Implantat ein Anker ist;
- Figur 26: zeigt eine schematische Darstellung einer Seitenansicht eines weiteren Beispiels eines chirurgischen Instruments, bei dem das Implantat eine Klammer ist;
- Figur 27: zeigt einen vergrösserten Ausschnitt eines vorderen Bereich des chirurgischen Instruments nach Figur 26;
- Figur 28: zeigt eine schematische Darstellung einer Draufsicht auf einen vorderen Bereich eines weiteren Beispiels eines chirurgischen Instruments
- Figur 29: zeigt eine schematische Darstellung einer geschnittenen Seitenansicht eines über eine Spindel expandierbaren Cages nach dem Stand der Technik;
- Figur 30: zeigt eine schematische Darstellung einer geschnittenen Seitenansicht eines Beispiels eines chirurgischen Instruments mit einem Cage als Implantat;
- Figur 31: zeigt eine schematische Darstellung einer Draufsicht des chirurgischen Instruments mit einem Cage als Implantat nach Figur 30;
- Figur 32: zeigt eine schematische Darstellung einer geschnittenen Seiteneinsicht eines weiteren Beispiels eines chirurgischen Instruments mit einem Cage als Implantat;
- Figur 33: zeigt eine schematische Darstellung einer geschnittenen Seitenansicht eines weiteren Beispiels eines chirurgischen Instrumentes mit einem Cage als Implantat in einem nicht expandierten Zustand;
- Figur 34: zeigt eine schematische Darstellung einer geschnittenen Seitenansicht des Beispiels des chirurgischen Instruments mit einem Cage als Implantat nach Figur 34, wobei der Cage in einem expandierten Zustand ist;
- Figur 35: zeigt eine schematische Darstellung einer geschnittenen Seitenansicht eines weiteren Beispiels eines chirurgischen Instruments mit einem Cage als Implantat;
- Figur 36: zeigt eine schematische Darstellung einer geschnittenen Seitenansicht eines weiteren Beispiels eines chirurgischen Instruments mit einem Cage als Implantat;
- Figur 37: zeigt eine schematische Darstellung einer geschnittenen Seitenansicht eines weiteren Beispiels eines chirurgischen Instruments mit einem Cage als Implantat.

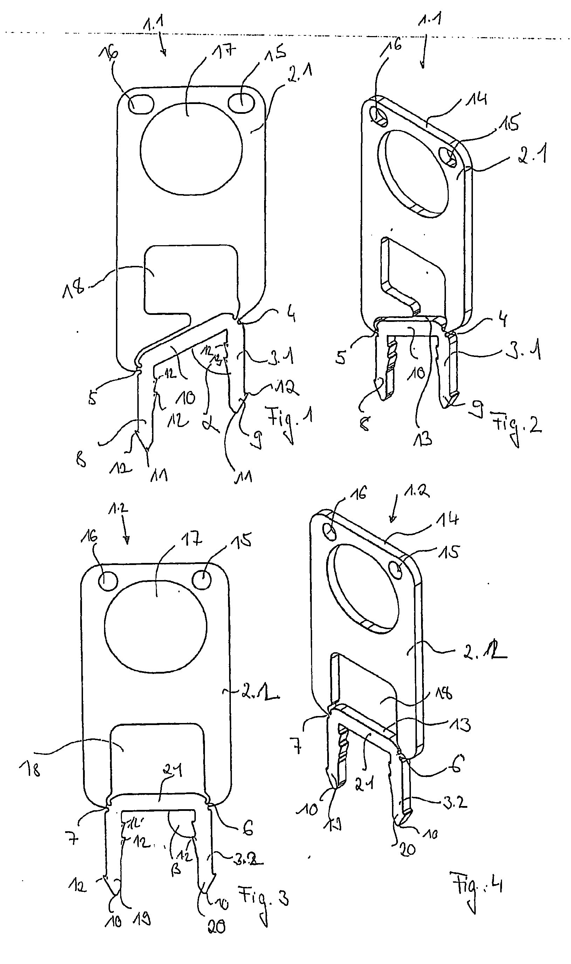

Die Figuren 1 und 2 offenbaren ein chirurgisches Instrument 1.1. Das chirurgische Instrument 1.1 umfasst einen Griffteil 2.1 und ein Fügeprofil 3.1. Das Fügeprofil 3.1 umfasst einen ersten Schenkel 8 und einen zweiten Schenkel 9. Das Griffteil 2.1 ist mit dem Fügeprofil 3.1 über eine erste Sollbruchstelle 4 und eine zweite Sollbruchstelle 5 verbunden.

Der erste Schenkel 8 und der zweite Schenkel 9 des Fügeprofils 3.1 sind über einen Steg 10 verbunden. Der Schenkel 9 und der Steg 10 des Fügeprofils 3.1 schliessen einen Winkel α ein. Im vorliegenden Ausführungsbeispiel der Figuren 1 und 2 beträgt der Winkel α 26°.

Die freien Enden der Schenkel 8 und 9 weisen jeweils eine Spitze 11 auf. Die Spitzen 11 sind geschliffen. Des weiteren umfasst jeder der Schenkel 8 und 9 eine Mehrzahl von Widerhaken 12.

Das Fügeprofil 3.1 umfasst eine Fügeprofilschlagfläche 13. Die Fügeprofilschlagfläche 13 befindet sich an einer Oberfläche des Stegs 13 des Fügeprofils 3.1.

Das Griffteil 2.1 umfasst eine Griffteilschlagfläche 14. Die Griffteilschlagfläche 14 befindet sich an einer Oberseite des Griffteils 2.1. Des weiteren umfasst das Griffteil 2.1 zwei Schablonenbohrungen 15 und 16. Zudem umfasst das Griffteil 2.1 eine Kreisausnehmung 17 und eine Rechteckausnehmung 18, wobei die Rechteckausnehmung 18 so ausgebildet ist, dass sie die Sollbruchstellen 5 und 4 zwischen dem Griffteil 2.1 und des Fügeprofils 3.1 ausbildet.

Das chirurgische Instrument 1.2 der Figuren 3 und 4 ist im wesentlichen analog zu dem chirurgischen Instrument 1.1 der Figuren 1 und 2 ausgebildet. Der wesentliche Unterschied ist, dass ein Fügeprofil 3.2 rechtwinklig ausgebildet ist, d.h., ein erster Schenkel 19 und ein zweiter Schenkel 20 des Fügeprofils 3.2 schliessen mit einem Steg 21 des Fügeprofils 3.2 einen Winkel β, der ein 90°Winkel ist, ein. Alle anderen Teile des chirurgischen Instruments 1.2 sind im wesentlichen analog zu den Bauteilen des chirurgischen Instruments 1.1 der Figuren 1 und 2 ausgebildet und erhalten deshalb gleiche Bezugszeichen. Es versteht sich, dass ein Griffteil 2.2 des chirurgischen Instruments 1.2 so von dem Griffteil 2.1 abweicht, dass es an das 90° Fügeprofil 3.2 angebunden werden kann.

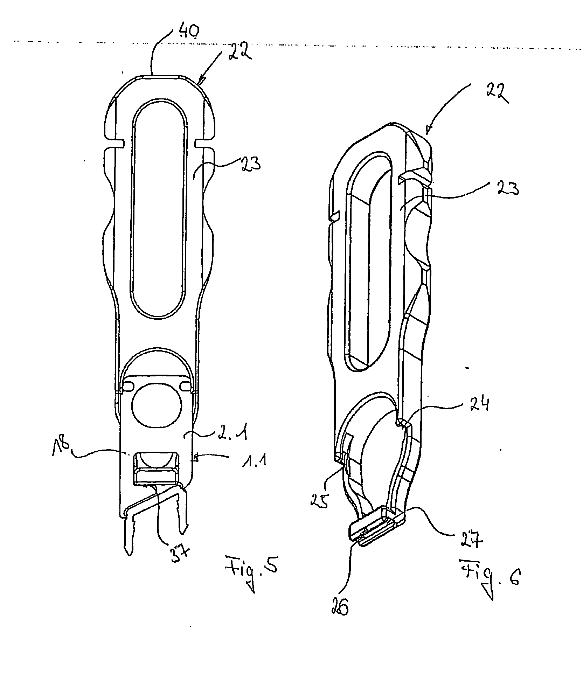

Die Figur 5 offenbart ein Einbringinstrument 22 mit dem chirurgischen Instrument 1.1. Das Einbringinstrument 22 umfasst, wie in den Figuren 5 und 6 dargestellt, einen Griff 23. An einer Oberseite des Griffs 23 umfasst das Einbringinstrument 22 eine Schlagfläche 40. Des weiteren umfasst das Einbringinstrument 22 eine erste Führungsnut 24 und eine zweite Führungsnut 25 für das Griffteil 2.1 bzw. 2.2.

Wie in Figur 6 dargestellt, umfasst das Griffteil 22 eine Auflagefläche 26 an einem Ende des Fügeprofils. Die Auflagefläche 26 ist geeignet zur Anlage an der Fügeprofilschlagfläche 13 oder der Griffschlagfläche 14 des chirurgischen Instruments 2.1 bzw. 2.2. Des weiteren umfasst die Auflagefläche 26 des Einbringinstruments 22 eine Führungsnut 27. Die Führungsnut 27 ist geeignet, eine Oberseite des Griffteils 2.1 oder 2.2 zu umgreifen und zu Führen.

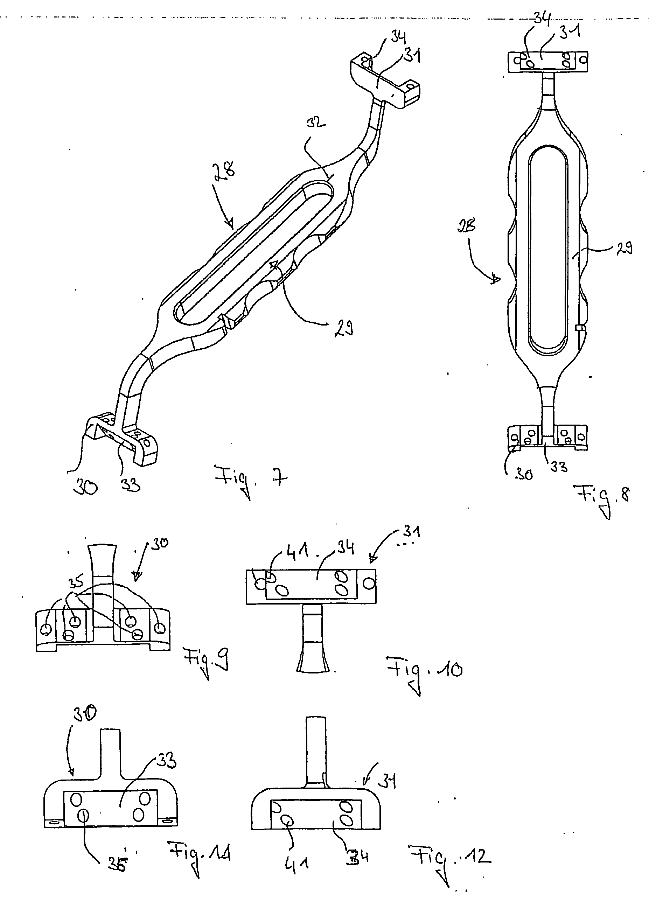

Die Figuren 7 und 8 zeigen eine Bohrschablone 28. Die Bohrschablone 28 umfasst einen Griff 29 zur guten Handhabung der Bohrschablone 28. An seinem ersten Ende umfasst die Bohrschablone 28 ein Funktionselement 30. An seinem zweiten Ende umfasst die Bohrschablone 28 ein Funktionselement 31. Die Funktionselemente 30 und 31 weisen jeweils eine Mehrzahl von Schablonenelementen und Führungselementen auf. Sowohl die Funktionseinheit 30, wie auch die Funktionseinheit 31 weisen jeweils eine Führungsfläche 33 bzw. 34 für das chirurgische Instrument 1.1 oder 1.2 auf.

Die Funktionseinheit 30 ragt so vom Griff 29 der Bohrschablone 28 ab, dass die Funktionseinheit 30 wenn eine Oberfläche 32 der Bohrschablone 28 vom behandelnden Patienten wegzeigt, am Körper des Patienten in Eingriff gebracht werden kann. Wenn die Oberseite 32 der Bohrschablone 28 zum Patienten hin zeigt, ist die Funktionseinheit 31 im Eingriff bzw. als Schablone verwendbar.

Die Figur 9 zeigt eine vergrösserte Draufsicht der Funktionseinheit 30 der Bohrschablone 28. Diese umfasst eine Mehrzahl von Bohrungen 35, die sich als Schablonen zum Einbringen von sogenannten 90° Fügeprofilen, also dem chirurgischen Instrument 1.2 eignen. Passend dazu offenbart die Figur 11 eine Unteransicht der Funktionseinheit 30, mit Bohrungen 36, die sich ebenfalls zum Einbringen des chirurgischen Instruments 1.2 eignet. Sowohl die Bohrungen 35, wie auch die Bohrungen 36 sind in einem rechten Winkel zu einer Oberfläche der Funktionseinheit 30 eingebracht. In der Figur 11 erscheinen die Bohrungen 36 konisch, weil sie in der abgeschrägten Führungsfläche 33 der Funktionseinheit 30 liegen.

Die Figur 10 zeigt eine Draufsicht der Funktionseinheit 31, die sich als Schablone für das chirurgische Instrument 1.1 eignet. Deshalb sind die Bohrungen 41 und im Winkel α, bevorzugt 26°, ausgebildet. Mit Hilfe der Funktionseinheit 31 der Bohrschablone 28 können Bohrungen im passenden Winkel für die Schenkel 8 und 9 des Fügeprofils 3.1 des chirurgischen Instruments 1.1 erzeugt werden

Die Funktionsweise der vorliegenden Erfindung ist folgende:

Die chirurgischen Instrumente 1.1. und 2.1 werden auf die gleiche Art und Weise implantiert. Im folgenden wird aus Gründen der Übersichtlichkeit nur die Implantation des chirurgischen Instruments 1.2 beschrieben.

Zum Implantieren des Fügeprofils 3.2 wird diese mit dem chirurgischen Instrument 1.2 in das Einbringinstrument 22 eingelegt. Dazu wird das Griffteil 2.2 des chirurgischen Instruments 1.2 in die Führungsnuten 24 und 25 des Einbringinstruments 22 geführt. Dabei greift die Anlagefläche 26 so in die Rechteckausnehmung 18 des Griffteils 2.2 ein, dass die Anlagefläche 26 in der Rechteckausnehmung 18 und an der Schlagfläche 13 des Fügeprofils 3.2 anliegt. Zur verbesserten Führung liegt das Griffteil 2.2 mit seiner Griffteilschlagfläche 14 in der Führungsnut 27 des Einbringinstruments 22.

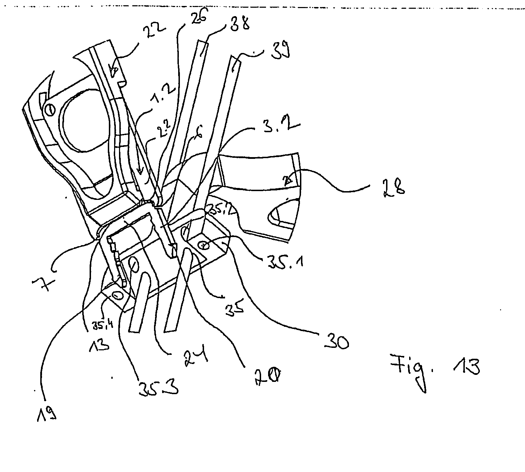

Figur 13 zeigt das chirurgische Instrument 1.2, das in dem Einbringinstrument 22 auf genommen ist, die Bohrschablone 28 und einen ersten Bohrdraht 38 und einen zweiten Bohrdraht 39. Die Bohrdrähte 38 und 39 sind bereits in Körpergewebe, bevorzugt Knochen, eingesetzt

Um das chirurgische Instrument 1.2 gut positionieren zu können, wird die Bohrschablone 28 mit ihren Öffnungen 35 über den ersten Bohrdraht 38 und den zweiten Bohrdraht 39 geführt.

Die freien Bohrungen 35.1 bis 35.4 sowie die Bohrungen, in denen die Bohrdrähte 38 und 39 eingesetzt sind, haben mehrere Zwecke. Es können weitere Bohrdrähte zur Fixierung der Knochenteile eingesetzt werden. Des weiteren dienen die Bohrungen 35.1 bis 35.4 auch zumindest teilweise als Schablonen zum Vorbohren von Öffnungen für die Schenkel 19 bzw. 20 des Fügeprofils 3.2. Zum Einsetzen des Fügeprofils 3.2 können die Bohrdrähte 38 und 39 zumindest teilweise aus dem Gewebe bzw. Knochen entnommen werden.

Um das Fügeprofil 3.2 in den Knochen einzutreiben, können Schläge auf die Schlagfläche 40 des Einbringinstruments 22 aufgebracht werden. Wenn das Fügeprofil 3.2 bis zu einer gewissen Tiefe eingebracht ist, kann über eine Dreh- und/oder Knickbewegung das Fügeprofil 3.2 an den Sollbruchstellen 6 und 7 von dem Griffteil 2.2 getrennt werden.

Es besteht weiter die Möglichkeit, das Fügeprofil 3.2 durch Schläge auf die Fügeprofilschlagfläche 13 weiter in das Gewebe bzw. den Knochen einzutreiben.

Alternativ kann das chirurgische Instrument in weiteren, nicht dargestellten Ausführungsbeispielen auch ohne das Einbringinstrument direkt in Gewebe, bevorzugt Knochen eingebracht werden. Durch das Griffteil ist das Fügeprofil relativ gut handhabbar, so dass die Möglichkeit besteht, dass ein Chirurg das chirurgische Instrument an dem Griffteil greift und das Fügeprofil über Schläge auf die Griffteilschlagfläche zumindest teilweise in das Gewebe eintreibt. Dann kann der Chirurg das Griffteil an den Sollbruchstellen von dem Fügeprofil trennen. Wenn nötig kann das Fügeprofil durch Schläge auf die Griffteilschlagfläche weiter in den Knochen eingetrieben werden.

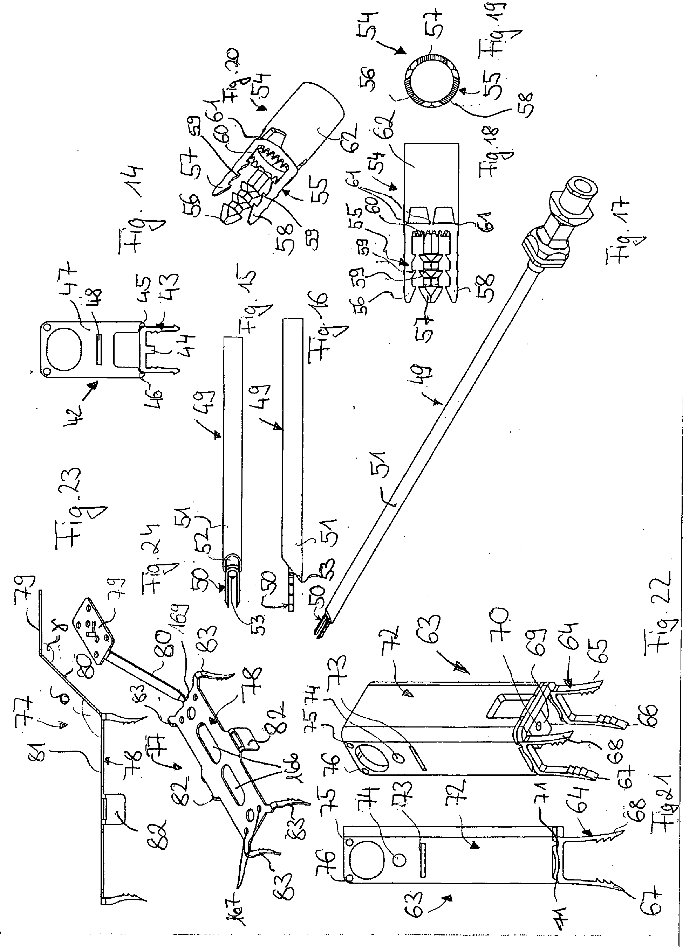

Figur 14 zeigt einen vorderen Bereich eines chirurgischen Instruments 42 mit einem Staple 43 als Implantat. Der Staple 43 weist einen Keil 44 auf. Der Keil 44 eignet sich zur Winkelkorrektur. Der Staple 43 ist über zwei Sollbruchstellen 45 und 46 mit einem Griffteil 47 verbunden. Das Griffteil weist einen Schlitz 48 auf. Der Schlitz 48 ist beispielsweise geeignet als Sägeschablone. Die Figuren 15, 16 und 17 zeigen ein weiteres Beispiel eines Instruments 49. Als Implantat ist ein Staple 50 vorgesehen. Der Staple 50 weist eine geringe Grösse, vorzugsweise kleiner als 4 mm auf. Desweitern umfasst das chirurgische Instrument 49 eine Kanüle 51. Das chirurgische Instrument aus Griffteil 52 und Staple 50 ist in der Kanüle 51 geführt. Die Kanüle 51 weist eine scharfe Kante 53 auf. Dadurch ergibt sich der Vorteil, dass sich das chirurgische Instrument 49 auch zum Schneiden eignet. Das Beispiel des chirurgischen Instruments 49 nach den Figuren 15, 16 und 17 eignet sich insbesondere zum perkutanen Fixieren von z.B. kleinen Fragmenten eines Knochens. Durch die Kanüle 51 kann der Staple 50 ohne grosse Beschädigung des umliegenden Gewebes eingebracht werden. Die Figuren 18, 19 und 20 zeigen ein weiteres Beispiel eines chirurgischen Instruments 54 mit einem Staple 55 als Implantat. Der Staple 55 ist aus einem Rohr hergestellt. Der Staple 55 weist drei Schenkel 56, 57 und 58, wie in Figur 19 erkennbar, auf. Der Staple 55 bzw. die Schenkel 56, 57 und 58 weisen Widerhaken 59 auf. Des Weiteren weist der Staple 55 in seinem oberen Bereich Spikes 60 auf.

Der Staple 55 ist über eine Mehrzahl von Sollbruchstellen 61 mit einem Griffteil 62 verbunden. Das Griffteil 62 ist rohrförmig ausgebildet. Dadurch ergibt sich der Vorteil, dass das chirurgische Instrument 54 aus einem Rohr hergestellt werden kann. Zum Ausschneiden der nicht benötigten Teile wird vorzugsweise ein Laserverfahren verwendet.

Die Figuren 21 und 22 zeigen ein weiteres Ausführungsbeispiel eines chirurgischen Instruments 63. Das chirurgische Instrument 63 umfasst einen Staple 64 mit vier Schenkeln 65, 66, 67 und 68. Des Weiteren umfasst der Staple 64 eine Platte 69 mit einer Befestigungsbohrung 70. Über eine Mehrzahl von Sollbruchstellen 71 ist der Staple 64 mit einem Griffteil 72 verbunden. Das Griffteil 72 weist einen Schlitz 73 auf, der als Sägeschablone dienen kann. Bohrungen 74 bis 76 in dem Griffteil 72 dienen als Bohrschablonen.

Die Figuren 23 und 24 zeigen ein weiteres Beispiel eines chirurgischen Instruments 77. Das chirurgische Instrument 77 umfasst als Implantat einen Staple 78 und als Griffteil eine Halteplatte 79. Die Halteplatte 79 ist über einen Verbindungssteg 80 mit dem Staple 78 verbunden. Die Halteplatte 79 ist in einem Winkel γ zu dem Verbindungssteg 80 angeordnet. Der Winkel α liegt bevorzugt zwischen 90° und 120°.

Der Verbindungssteg 80 schliesst mit einer Kompressionsplatte 81 des Staples 78 einen Winkel δein. Der Verbindungssteg 80 ist über eine Sollbruchstelle 169 mit der Kompressionsplatte 81 verbunden. Der Winkel δ liegt vorzugsweise zwischen 90° und 170°. Der Staple 78 umfasst eine Kompressionsplatte 81.Diese weist einen seitlichen Keil 82 zur Winkelkorrektur auf.

Des Weiteren umfasst der Staple 78 an jeder Ecke der Kompressionsplatte 81 einen Schenkel 83. Analog zu den bereits vorhergehend beschriebenen Beispielen umfasst die Haltelatte 79 Öffnungen 166 und Schlitze 167, die als Bohrschablonen dienen.

Die Funktionsweise der Beispiele und des Ausführungsbeispiels der Figuren 14 bis 23 ist folgende:

Der Staple 43, 50, 55, 64 oder 78 wird mittels des Griffteils 47, 52, 62, 72 oder 79 positioniert und dann in das Gewebe, vorzugsweise Knochenmaterial eingetrieben. Wenn der Staple 43, 50, 55, 64 oder 78 richtig positioniert ist, wird über die Sollbruchstellen 45, 46, 61, 71,169, das Griffteil 47, 52, 62, 72 oder 79 vom Staple 43, 50, 55, 64 oder 78 getrennt.

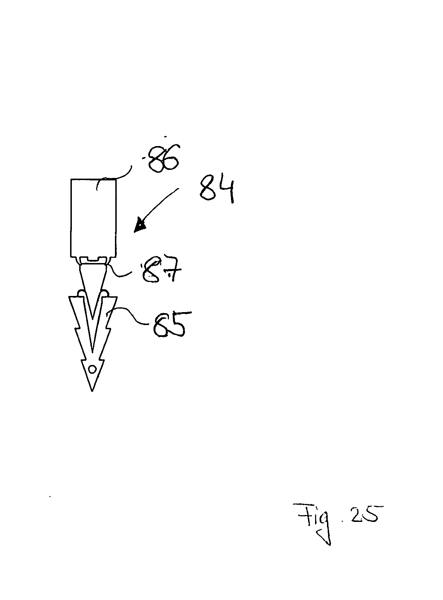

Figur 25 zeigt ein weiteres chirurgisches Instrument 84. Das chirurgische Instrument 84 umfasst als Implantat einen Anker 85 und ein Griffteil 86. Der Anker 85 ist über eine Sollbruchstelle 87 mit dem Griffteil 86 verbunden. Im dargestellten Beispiel ist die Sollbruchstelle 87 ringförmig ausgebildet. Die Funktionsweise ist analog zu den vorgehend beschriebenen Beispielen und
Ausführungsbeispielen von chirurgischen Instrumenten.

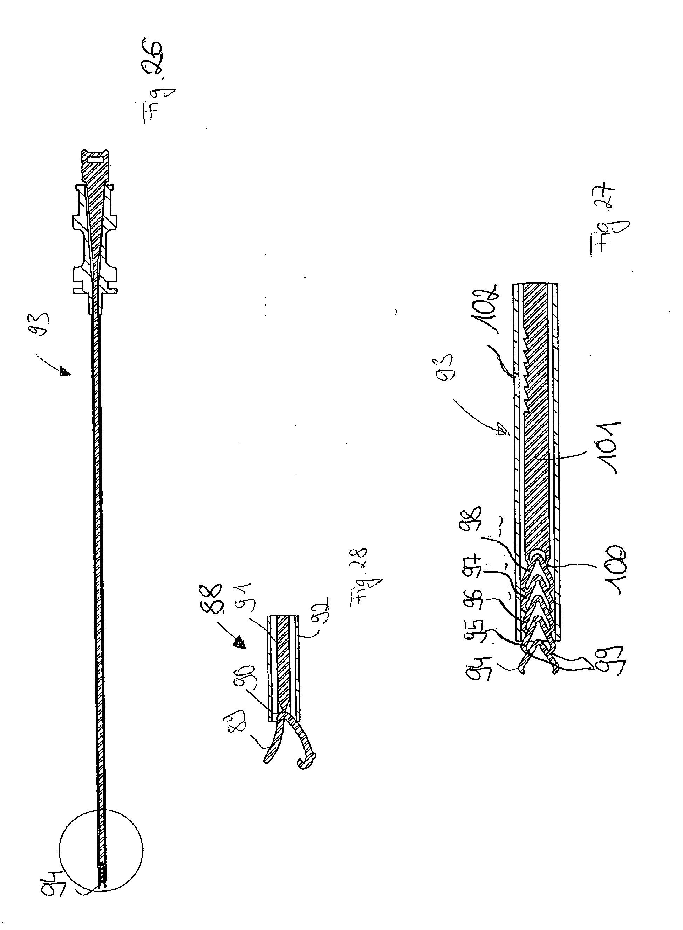

In weiteren nicht dargestellten Beispielen ist das chirurgische Instrument 84 in einem Führungsrohr, insbesondere einer Kanüle geführt. Figur 26 zeigt ein weiteres Beispiel eines chirurgischen Instruments 93.

Die Figur 27 zeigt einen vergrösserten Ausschnitt eines vorderen Bereichs des chirurgischen Instruments 93 Bei diesem sind als Implantat eine Mehrzahl von Klammern 94, 95, 96, 97 und 98 vorgesehen. Die Klammer 94 ist über eine Sollbruchstelle bzw. über zwei Sollbruchstellen 99 mit der Klammer 95 verbunden. Analog ist die Klammer 95 mit der Klammer 96 verbunden. Die Klammer 98 ist über eine Sollbruchstelle 100 mit einem Griffteil 101 verbunden. Das Griffteil 101 und die Mehrzahl der Klammern 94 bis 98 sind in einer Kanüle 102 geführt. Das Griffteil 101 kann über eine Kupplung mit einem nicht näher gezeigten Instrumentengriff verbunden sein. Grundsätzlich kann das Griffteil 101 jeweils einstückig an den Instrumentengriffe angeformt sein. Bei dem in Figur 27 dargestellten Beispiel können die Klammer 94, 95, 96, 97 und 98 nacheinander wie aus einer Art Magazin ausgebracht werden.

Die Figur 28 zeigt eine vergrösserte Draufsicht auf einen vorderen Bereich eines chirurgischen Instruments 88. Eine Klammer 89 ist über eine Sollbruchstelle 90 mit einem Griffteil 91 verbunden. Das Griffteil 91 und die Sollbruchstelle 90 sind in einer Kanüle 92 geführt. Das Griffteil 91 kann über eine Kupplung mit einem nicht näher gezeigten Instrumentengriff verbunden sein. Grundsätzlich kann dann der Griffteil 91 einstückig an den Instrumentengriff angeformt sein.

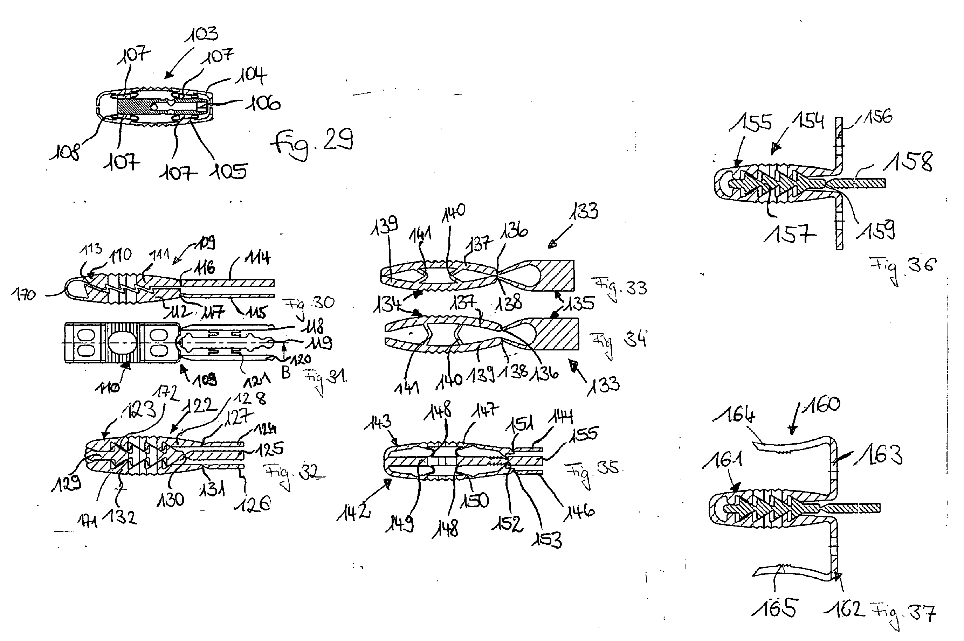

Die Figur 29 zeigt einen Cage 103 als Implantat nach dem Stand der Technik. Der Cage 103 umfasst eine Oberschale 104 und eine Unterschale 105. Über eine Anschlussstelle 106 für eine nicht näher gezeigte Spindel kann der Cage 103 expandiert werden. Durch Betätigen der Anschlussstelle 106 mit einer Spindel werden die Keile 107 verschoben. Dadurch werden die Keile 107 verstellt, so dass der Cage 103 expandiert wird. Die Oberschale 104 ist mit der Unterschale 105 neben den Keilen 107 auch über flexible Bänder 108 verbunden.

Die Figuren 30 und 31 zeigen ein chirurgisches Instrument 109 mit einem Cage 110 als Implantat. Der Cage 110 umfasst eine Oberschale 111 und eine Unterschale 112. Die Oberschale 111 und auch die Unterschale 112 sind an den einander zugewandten Flächen wie eine Mehrzahl von kleinen Keilen, die eine Zick-Zackform ausbilden und über ein flexibles Band 113 miteinander verbunden. Das flexible Band 113 ist zwischen einer der keilörmig und einander zugewandten Innenseiten des der Oberschale 111 und der Unterschale 112 angeordnet. Vorzugsweise ist das Band 113 aus einem elastischen Material hergestellt.

Zur Verbindung des elastischen Bands 113 mit dem Cage ist das Band vorzugsweise einstückig mit dem Cage hergestellt bzw. eingespritzt. Alternativ kann da Band auch an den Cage genietet oder geklebt sein. Zudem sind die Oberschale 111 und die Unterschale 112 über einen Bogen 117 einstückig miteinander verbunden.

Dadurch, dass die Oberschale 111 mit der Unterschale 112 über mindestens ein flexibles Band 113 verbunden ist, ergibt sich der Vorteil, dass keine weiteren aufwändigen Haltemechanismen wie Gelenke oder Scharniere eingesetzt werden müssen.

In weiteren nicht dargestellten Beispielen kann die Unter- bzw. die Oberschale auch mindestens eine abtrennbare Verbindung aufweisen oder weitere abtrennbare Verbindungen besitzen.

Als Griffteil umfasst das chirurgische Instrument 109 zwei Stäbe 114 und 115. Der Stab 114 ist über eine Sollbruchstelle 116 mit der Oberschale 111 des Cages 110 verbunden. Der Stab 115 ist über eine Sollbruchstelle 117 mit der Unterschale 112 des Cages 110 verbunden. Der obere Stab 114 ist dabei nochmals in drei Streifen 118, 119 und 120 unterteilt. Die Streifen können je nach Breite und Stabilität unterschiedliche Aufgaben und Funktionen wie beispielsweise Navigieren, Steuern, Expandieren und/oder Kuppeln erfüllen. Die einzelnen Streifen 118 bis 120 sind über flexible Bänder 121 miteinander verbunden. Dadurch wird verhindert, dass beim Abbrechen der Streifen bzw. Stäbe 114, 115, 118, 119, 120 diese das Gewebe verletzen.

Die Funktionsweise des chirurgischen Instruments ist folgende:

Über die Stäbe 114 und 115 wird der Cage 110 korrekt positioniert und gehalten. Dann wird über einen Zug und/oder Druck an den Stäben 115 und 115 der Cage 110 expandiert bzw. die Oberschale 111 von der Unterschale 112 beabstandet. Über die Streifen 118 bis 120 ist eine weiter Ausrichtung des Cages 110 und der Oberschale 111 zu der Unterschale112 möglich. Wenn der Cage 110 richtig sitzt, werden die Stäbe 114 und 115 an den Sollbruchstellen 116 und 117 von dem Cage 110 getrennt.

Die Figur 32 zeigt ein weiteres Beispiel eines chirurgischen Instruments 122. Das chirurgische Instrument 122 umfasst als Implantat einen Cage 123 und als Griffteil drei Stäbe 124, 125 und 126. Der Cage 123 umfasst als Verbindungselement eine Keilkette 129. Zusätzlich ist die Keilkette 129 über ein flexibles Band 171 mit der Unterschale 132 und über ein flexibles Band 172 mit der Oberschale 128 verbunden. Der Stab 124 ist über eine Sollbruchstelle 127 mit einer Oberschale 128 des Cages 123 verbunden. Der Stab 125 ist mit der Keilkette 129 über eine Sollbruchstelle 130 verbunden. Der Stab 126 ist über eine Sollbruchstelle 131 mit der Unterschale 132 des Cages 123 verbunden.

Die Funktionsweise des chirurgischen Instruments 122 ist folgende:

Über die Stäbe 124 und 126 wird der Cage 123 korrekt positioniert und gehalten. Dann wird über einen Zug an dem Stab 125 der Cage 123 expandiert bzw. die Oberschale 128 von der Unterschale 132 beabstandet. Wenn der Cage 123 richtig sitzt, werden die Stäbe 124 bis 126 an den Sollbruchstellen 127, 130 und 131 von dem Cage 123 getrennt.

Die Figuren 33 und 34 zeigen ein weiteres Beispiel eines chirurgischen Instruments 133. Das chirurgische Instrument 133 umfasst als Implantat einen Cage 134 und als Griffteil eine Zug- oder Druckstange 135. Die Zug-/Druckstange 135 ist über eine Sollbruchstelle 136 mit einer Oberschale 137 des Cages 134 verbunden. Die Zug-/Druckstange 135 ist über eine Sollbruchstelle 138 mit einer Unterschale 139 des Cages 134 verbunden. Die Oberschale 137 und die Unterschale 139 des Cages 134 sind über einen ersten Steg 140 und einen zweiten Steg 141 direkt miteinander verbunden. Dadurch ergibt sich der Vorteil, dass der Cage 134 einstückig hergestellt werden könnte. Die Stege 140 und 141 dienen dem Expandieren des Cages 134 über Zug und/oder Druck, wobei die Stegausrichtung auch in eine Richtung verlaufen kann. Zur Ausrichtung des Cages dient die Zug-/Druckstange 135.

In weiteren nicht dargestellten Beispielen kann zum Ausrichten des Cages auch ein zusätzliches separates Instrument eingesetzt werden.

Die Figur 35 zeigt ein weiteres Beispiel eines chirurgischen Instruments 142. Das chirurgische Instrument 142 umfasst als Implantat einen Cage 143 und als Griffteil drei Stangen 144, 145 und 146. Eine Oberschale 147 des Cages 143 ist über flexible Bänder 148 mit einem Mittelsteg 149 verbunden. Eine Unterschale 150 ist analog über flexible Bänder 148 mit dem Mittelsteg 149 verbunden. Über die Stangen 144 bis 146 wird der Cage 143 positioniert. Durch Zug und/oder Druck mit der Stange 145 auf den Mittelsteg 149 werden die Oberschale 147 und die Unterschale 150 voneinander expandiert. Wenn der Cage 143 die richtige Öffnungsweite aufweist und richtig positioniert ist, werden die Stäbe 144, 146 und 146 an entsprechenden Sollbruchstellen 151, 152 und 153 vom Cage 143 getrennt.

Figur 36 zeigt ein weiteres Beispiel eines chirurgischen Instruments 154. Das chirurgische Instrument 154 umfasst als Implantat einen Cage 155. Der Cage 155 umfasst eine integrierte Versteifungsplatte 156. Im vorliegenden Beispiel wird der Cage 155 über eine Keilkette 157 verstellt.

Als Griffteil umfasst das chirurgische Instrument 154 einen Stab 158, der über eine Sollbruchstelle 159 mit der Keilkette 157 verbunden ist. In weiteren nicht dargestellten Beispielen können alle bis jetzt genannten Cageformen mit einer Versteifungsplatte kombiniert sein. Die Figur 37 offenbart ein weiteres Beispiel eines chirurgischen Instruments 160. Das chirurgische Instrument 160 umfasst als Implantat einen Cage 161 mit einem integrierten Staple 162. Der Staple 162 umfasst eine Stapleplatte 163 und zwei Stapleschenkel 164 und 165. Ein Griffteil 168 ist analog zu den vorgehenden beschriebenen Ausführungen ausgebildet.

Bei einem chirurgischen Instrument soll das Griffteil 2.1, 2.2 vorzugsweise eine Bohrung aufweisen.

Bei einem chirurgischen Instrument soll das Griffteil vorzugsweise ein Stab 114, 115 sein.

Bei chirurgischen Instrument sollen vorzugsweise eine Mehrzahl von Klammern 94, 95, 96, 97, 98 mit dem Griffteil 101 verbunden sein.

Bei einem chirurgischen Instrument sollen vorzugsweise eine Mehrzahl von Klammern 94, 95, 96, 97, 98 miteinander verbunden sein.

Bei einem chirurgischen Instrument soll der Cage 110, 123, 134, 143, 155, 161 vorzugsweise eine Oberschale 111, 128, 137, 147 eine Unterschale 112, 132, 139, 150 und eine Verbindungseinrichtung 113, 129, 141, 140, 149, 157 umfassen.

Bei einem chirurgischen Instrument soll das Griffteil 114, 124, 135, 144 vorzugsweise über eine Sollbruchstelle 116, 127, 136, 151 mit der Oberschale 111, 128, 137, 147 verbunden sein.

Bei einem chirurgischen Instrument soll das Griffteil 115, 126, 135, 146 vorzugsweise über eine Sollbruchstelle 117, 131, 138, 153 mit der Unterschale 112 132, 139, 159 verbunden sein.

Bei einem chirurgischen Instrument soll das Griffteil 125, 135, 145, 158 vorzugsweise über eine Sollbruchstelle 130, 136, 138, 153, 159 mit der Verbindungseinrichtung 129, 141, 140, 149, 157 verbunden sein.

Ein Einbringinstrument soll vorzugsweise eine Schlagfläche 40 umfassen.

Ein Einbringinstrument, vorzugsweise mit einer Auflagefläche, die mit der Schlagfläche des Fügeprofils 3.1, 3.2 und/oder der Schlagfläche 14 des Griffteils 2.1, 2.2 in Wirkverbindung bringbar ist.

Bei einem Einbringinstrument soll die Auflagefläche vorzugsweise eine Führungsnut 24, 25 aufweisen.

Ein Bohrschablone zum Einbringen eines chirurgischen Instruments 1.1, 1.2, vorzugsweise mit einer Schablone, geeignet zum Einbringen von Bohrungen, zur Fixierung des Knochens

Eine Bohrschablone zum Einbringen eines chirurgischen Instruments, vorzugsweise mit einer Führungsfläche 33, 34 zum Führen des Fügeprofils 3.1, 3.2 und/oder des Griffteils 2.1, 2.2 beim Einbringen des Fügeprofils 3.1, 3.2.

Eine Bohrschablone zum Einbringen eines chirurgischen Instruments 1.1, 1.2, vorzugsweise mit einer Schablone, geeignet zum Einbringen von Bohrungen, für das Fügeprofil und zur Fixierung des Knochens auch über min. eine Sollbruchstelle am Griff integriert und abbrechbar verbunden ist.

Eine Bohrschablone zum Einbringen eines chirurgischen Instruments 1.1, 1.2, vorzugsweise mit einer abbrechbare Schablone, die zumindest eine Führungsnut zum Führen des Fügeprofils beim Einbringen des Fügeprofils 3.1, 3.2 unterstützt.

**Bezugszeichenliste**

| | | | | | |
|---|---|---|---|---|---|
| 1 | Chirurgisches Instrument | 34 | Führungsfläche | 67 | Schenkel |
| 2 | Griffteil | 35 | Bohrungen | 68 | Schenkel |
| 3 | Fügeprofil | 36 | Bohrungen | 69 | Platte |
| 4 | Sollbruchstelle | 37 | Gegenfläche | 70 | Befestigungsbohrung |
| 5 | Sollbruchstelle | 38 | Bohrdraht | 71 | Sollbruchstelle |
| 6 | Sollbruchstelle | 39 | Bohrdraht | 72 | Griffteil |
| 7 | Sollbruchstelle | 40 | Schlagfläche | 73 | Schlitz |
| 8 | Schenkel | 41 | Bohrung | 74 | Bohrungen |
| 9 | Schenkel | 42 | chir. Instrument | 75 | Bohrungen |
| 10 | Steg | 43 | Staple | 76 | Bohrungen |
| 11 | Spitze | 44 | Keil | 77 | chir. Instrument |
| 12 | Widerhaken | 45 | Sollbruchstellen | 78 | Staple |
| 13 | Fügeprofilschlagfläche | 46 | Sollbruchstellen | 79 | Halteplatte |
| 14 | Griffteilschlagfläche | 47 | Griffteil | 80 | Verbindungssteg |
| 15 | Schablonenbohrung | 48 | Schlitz | 81 | Kompressionsplatte |
| 16 | Schablonenbohrung | 49 | chir. Instrument | 82 | Keil |
| 17 | Kreisausnehmung | 50 | Staple | 83 | Schenkel |
| 18 | Rechteckausnehmung | 51 | Kanüle | 84 | chir. Instrument |
| 19 | Schenkel | 52 | Griffteil | γ | Winkel |
| 20 | Schenkel | 53 | Kante | δ | Winkel |
| 21 | Steg | 54 | chir. Instrument | 85 | Anker |
| 22 | Einbringinstrument | 55 | Staple | 86 | Griffteil |
| 23 | Griff | 56 | Schenkel | 87 | Sollbruchstelle |
| 24 | Führungsnut | 57 | Schenkel | 88 | chir. Instrument |
| 25 | Führungsnut | 58 | Schenkel | 89 | Klammer |
| 26 | Auflagefläche | 59 | Widerhaken | 90 | Sollbruchstelle |
| 27 | Führungsnut | 60 | Spike | 91 | Griffteil |
| 28 | Bohrschablone | 61 | Sollbruchstelle | 92 | Kanüle |
| 29 | Griff | 62 | Griffteil | 93 | chir. Instrument |
| 30 | Funktionseinheit | 63 | chir. Instrument | 94-98 | Klammern |
| 31 | Funktionseinheit | 64 | Staple | 99 | Sollbruchstellen |
| 32 | Oberflächen | 65 | Schenkel | 100 | Sollbruchstelle |
| 33 | Führungsfläche | 66 | Schenkel | 101 | Griffteil |
| 102 | Kanüle | 103 | Cage | 104 | Oberschale |
| 105 | Unterschale | 106 | Anschlussstelle | 107 | Keile |
| 108 | Flexible Bänder | 109 | chir. Instrument | 110 | Cage |
| 111 | Oberschale | 112 | Unterschale | 113 | flexible Bänder |
| 114 | Griffteil, Stab | 115 | Stab | 116 | Sollbruchstelle |
| 117 | Sollbruchstelle | 118-120 | Streifen | 121 | flexible Bänder |
| 122 | chir. Instrument | 123 | Cage | 124 - 126 | drei Stäbe |
| 127 | Sollbruchstelle | 128 | Oberschale | 129 | Keilkette |
| 130 | Sollbruchstelle | 131 | Sollbruchstelle | 132 | Unterschale |
| 133 | chir. Instrument | 134 | Cage | 135 | Zug- o. Druckstange |
| 136 | Sollbruchstelle | 137 | Oberschale | 138 | Sollbruchstelle |
| 139 | Unterschale | 140 | erster Steg | 141 | zweiter Steg |
| 142 | chir. Instrument | 143 | Cage | 144 - 146 | drei Stangen |
| 147 | Oberschale | 148 | Flexible Bänder | 149 | Mittelsteg |
| 150 | Unterschale | 151 - 153 | Sollbruchstellen | 154 | chir. Instrument |
| 155 | Cage | 156 | Versteifungsplatte | 157 | Keilkette |
| 158 | Stab | 159 | Sollbruchstelle | 160 | chir. Instrument |
| 161 | Cage | 162 | Staple | 163 | Stapleplatte |
| 164 - 165 | Stapleschenkel | 166 | Öffnungen | 167 | Bohrungen |
| 168 | Griffteil | 169 | Sollbruchstelle | | |

## Patentansprüche

1. Chirurgisches Instrument (1.1, 1.2), mit einem Implantat und einem Griffteil (2.1, 2.2) und einer Sollbruchstelle (4, 5, 6, 7), die das Implantat und das Griffteil (2.1, 2.2) verbindet, wobei das Implantat ein Fügeprofil (3.1, 3.2) ist, wobei das Fügeprofil (3.1, 3.2) einen ersten Schenkel (8) und einen zweiten Schenkel (9) umfasst, wobei das Griffteil (2.1, 2.2) mit dem Fügeprofil (3.1, 3.2) über eine erste Sollbruchstelle (4, 6) und eine zweite Sollbruchstelle (5, 7) verbunden ist, wobei der erste Schenkel (8, 19) und der zweite Schenkel (9, 20) des Fügeprofils (3.1) über einen Steg (10, 21) miteinander verbunden sind und die freien Enden der Schenkel (8, 9, 19, 20) jeweils eine Spitze (11) aufweisen,
wobei die Spitzen (11) geschliffen sind, wobei das Griffteil (2.1, 2.2) eine Rechteckausnehmung (18) umfasst, wobei die Rechteckausnehmung (18) so ausgebildet ist, dass sie die Sollbruchstellen (4, 5, 6, 7) zwischen dem Griffteil (2.1, 2.2) und dem Fügeprofil (3.1, 3.2) ausbildet
**dadurch gekennzeichnet,**
**dass** jeder der Schenkel (8, 9) eine Mehrzahl von Widerhaken (12) aufweist, wobei das Griffteil (2.1, 2.2) zwei Schablonenbohrungen (15, 16) und eine Kreisausnehmung (17) aufweist.

2. Chirurgisches Instrument (49, 84, 93, 88) nach Anspruch 1, **dadurch gekennzeichnet, dass** das Instrument ein Führungsrohr (51, 92, 102), insbesondere eine Kanüle umfasst.

3. Chirurgisches Instrument nach Anspruch 1 oder 2, **dadurch gekennzeichnet, dass** das Implantat eine Staple (43, 50, 55, 64, 78) ist.

4. Chirurgisches Instrument nach einem der vorhergehenden Ansprüche, **gekennzeichnet durch** eine Schlagfläche, wobei die Schlagfläche (13) nach dem Abbrechen des Griffteils (2.1, 2.2) an dem Fügeprofil (3.1, 3.2) entsteht.

5. Chirurgisches Instrument nach einem der vorhergehenden Ansprüche, **gekennzeichnet durch** einen Aufbau an dem Fügeprofil (3.1, 3.2), wobei der Aufbau eine Schlagfläche (13) ausbildet.

6. Chirurgisches Instrument nach einem der vorhergehenden Ansprüche, **dadurch gekennzeichnet, dass** das Griffteil (2.1, 2.2) eine Schlagfläche (14) aufweist.

7. Chirurgisches Instrument nach Anspruch 6, **dadurch gekennzeichnet, dass** die Schlagfläche (14) des Griffteils (2.1, 2.2) ein Aufbau ist.

## Claims

1. A surgical instrument (1.1, 1.2), with an implant and a grip part (2.1, 2.2) and a predetermined breaking point (4, 5, 6, 7) which connects the implant and the grip part (2.1, 2.2), wherein the implant is a joining profile (3.1, 3.2), wherein the joining profile (3.1, 3.2) comprises a first leg (8) and a second leg (9), wherein the grip part (2.1, 2.2) is connected to the joining profile (3.1, 3.2) via a first predetermined breaking point (4, 6) and a second predetermined breaking point (5, 7), wherein the first leg (8, 19) and the second leg (9, 20) of the joining profile (3.1) are connected together via a crosspiece (10, 21) and the free ends of the legs (8, 9, 19, 20) have a tip (11) in each case, wherein the tips (11) are ground, wherein the grip part (2.1, 2.2) comprises a rectangular cutout (18), wherein the rectangular cutout (18) is formed such that it forms the predetermined breaking points (4, 5, 6, 7) between the grip part (2.1, 2.2) and the joining profile (3.1, 3.2),
**characterised in that**
each of the legs (8, 9) has a plurality of barbs (12), the grip part (2.1, 2.2) having two template bores (15, 16) and a circular cutout (17).

2. A surgical instrument (49, 84, 93, 88) according to Claim 1, **characterised in that** the instrument comprises a guide tube (51, 92, 102), in particular a cannula.

3. A surgical instrument according to Claim 1 or 2, **characterised in that** the implant is a staple (43, 50, 55, 64, 78).

4. A surgical instrument according to one of the preceding claims, **characterised by** a striking surface, the striking surface (13) being produced on the joining profile (3.1, 3.2) once the grip part (2.1, 2.2) has been broken off.

5. A surgical instrument according to one of the preceding claims, **characterised by** a structure on the joining profile (3.1, 3.2), the structure forming a striking surface (13).

6. A surgical instrument according to one of the preceding claims, **characterised in that** the grip part (2.1, 2.2) has a striking surface (14).

7. A surgical instrument according to Claim 6, **characterised in that** the striking surface (14) of the grip part (2.1, 2.2) is a structure.

## Revendications

1. Instrument chirurgical (1.1, 1.2), avec un implant et une partie de préhension (2.1, 2.2) et un point de rupture de consigne (4, 5, 6, 7) qui relie l'implant et la partie de préhension (2.1, 2.2), l'implant étant un profilé assemblé (3.1, 3.2), le profilé assemblé (3.1, 3.2) comprenant une première branche (8) et une deuxième branche (9), la partie de préhension (2.1, 2.2) étant reliée au profilé assemblé (3.1, 3.2) par l'intermédiaire d'un premier point de rupture de consigne (4, 6) et d'un deuxième point de rupture de consigne (5, 7), la première branche (8, 19) et la deuxième branche (9, 20) du profilé assemblé (3.1) étant connectées l'une à l'autre par l'intermédiaire d'une bretelle (10, 21) et les extrémités libres des branches (8, 9, 19, 20) présentant, chacune, une pointe (11), les pointes (11) étant polies, la partie de préhension (2.1, 2.2) comportant un évidement rectangulaire (18), l'évidement rectangulaire (18) étant réalisé de sorte que les points de rupture de consigne (4, 5, 6, 7) soient réalisés entre la partie de préhension (2.1, 2.2) et le profilé assemblé (3.1, 3.2),
**caractérisé par le fait**
**que** chacune des branches (8, 9) présente une pluralité de barbes (12), la partie de préhension (2.1, 2.2) présentant deux alésages ovales (15, 16) et un évidement circulaire (17).

2. Instrument chirurgical (49, 84, 93, 88) selon la revendication 1, **caractérisé par le fait que** l'instrument comporte un tube de guidage (51, 92, 102), en particulier une canule.

3. Instrument chirurgical selon la revendication 1 ou 2, **caractérisé par le fait que** l'implant est une agrafe (43, 50, 55, 64, 78).

4. Instrument chirurgical selon l'une des revendications précédentes, **caractérisé par** une face de frappe, la face de frappe (13) étant créée au profilé assemblé (3.1, 3.2) après la cassure de la partie de préhension (2.1, 2.2).

5. Instrument chirurgical selon l'une des revendications précédentes, **caractérisé par** une structure sur le profilé assemblé (3.1, 3.2), la structure constituant une surface de frappe (13).

6. Instrument chirurgical selon l'une des revendications précédentes, **caractérisé par le fait que** l'élément de préhension (2.1, 2.2) présente une surface de frappe (14).

7. Instrument chirurgical selon la revendication 6, **caractérisé par le fait que** la surface de frappe (14) de la partie de préhension (2.1, 2.2) est une structure.
